**Europäisches Patentamt**

**European Patent Office**

**Office européen des brevets**

(19)

(11) Veröffentlichungsnummer: **0 057 890 B1**

(12)
# EUROPÄISCHE PATENTSCHRIFT

(45) Veröffentlichungstag der Patentschrift:
**23.01.85**

(51) Int. Cl.⁴: **C 07 D 213/133,** C 07 D 213/09, C 07 D 207/323

(21) Anmeldenummer: **82100681.4**

(22) Anmeldetag: **01.02.82**

(54) **Verfahren zur Herstellung von Pyridinen oder Pyrrolen aus alpha,omega-Dinitrilen.**

(30) Priorität: **11.02.81 DE 3104765**

(43) Veröffentlichungstag der Anmeldung:
**18.08.82 Patentblatt 82/33**

(45) Bekanntmachung des Hinweises auf die Patenterteilung:
**23.01.85 Patentblatt 85/4**

(84) Benannte Vertragsstaaten:
**AT BE CH DE FR GB IT LI NL**

(56) Entgegenhaltungen:
**DE - A - 2 514 004**
**DE - A - 2 519 529**
**DE - C - 699 032**
**DE - C - 942 990**

(73) Patentinhaber: **BASF Aktiengesellschaft, Carl-Bosch-Strasse 38, D-6700 Ludwigshafen (DE)**

(72) Erfinder: **Rebafka, Walter, Dr., Schuetzenstrasse 4, D-6904 Eppelheim (DE)**
Erfinder: **Heilen, Gerd, Dr., Schifferstadter Strasse 1 b, D-6702 Speyer (DE)**
Erfinder: **Halbritter, Klaus, Dr., Schwarzwaldstrasse 19, D-6800 Mannheim (DE)**
Erfinder: **Franzischka, Wolfgang, Dr., Franz-Lind-Strasse 1, D-6713 Freinsheim (DE)**

## Beschreibung

Es ist bekannt, Pyridin aus Glutarsäuredinitril in zwei Schritten herzustellen, indem man in einer Druckreaktion in flüssiger Phase an Nickel- oder Kobaltkatalysatoren zunächst das entsprechende Diamin herstellt; als Nebenprodukt wird in wechselnder Menge Piperidin erhalten, dessen Bildung auch durch gewisse Maßnahmen begünstigt werden kann (Freidlin et al Ber. Akad. Wiss. UdSSR 1962, S. 625 ff; DE-AS 2 514 004). Sowohl Pentamethylendiamin als auch Piperidin können in der Gasphase zu Pyridin dehydriert werden.

Bekannt ist auch $\alpha$, $\omega$-Dinitrile in der Flüssig- oder Gasphase zu Diaminen bzw. Aminonitrilen umzusetzen; nach der BE-PS 751 254 dient Eisen(oxid) als Katalysator, während die südafrikanische Patentanmeldung 39 237/67 die Verwendung von Nickel empfiehlt.

Die besondere Problematik der einstufigen Umsetzung von Dinitrilen zu aromatischen bzw. quasiaromatischen heterocyclischen Ringsystemen beruht darauf, daß Dinitrile thermisch verhältnismäßig labil sind, während die Dehydrierung der intermediär gebildeten (an sich stabilen) Diamine relativ scharfe Bedingungen erfordert.

Die Aufgabe der Erfindung, für dieses Problem eine Lösung zu finden, ergibt sich aus diesem Sachverhalt.

Es wurde nun gefunden, daß sich Dinitrile entsprechenden Kohlenstoffgerüsts unmittelbar in Pyridin, Pyrrol bzw. deren Substitutionsprodukte überführen lassen, wenn man sie in der Gasphase in Gegenwart eines hydrierenden, vorzugsweise ein Platinmetall enthaltenden Edelmetall-Katalysators mit Wasserstoff umsetzt.

Als Hydrierkatalysatoren werden bevorzugt solche mit Palladium oder Platin als wirksame Bestandteile verwendet.

In manchen Fällen hat sich die Verwendung zusätzlicher Metalle bewährt. So wird Palladium z. B. nach der Empfehlung der DE-OS 2 839 135 mit Vanadin und/oder Kupfer versetzt.

Günstig ist die Verwendung solcher Katalysatoren, die eine Trägersubstanz aufweisen, wie sie für das jeweilige Wirkmetall üblich sind. Besonders häufig werden als Träger Aluminiumoxid in verschiedener Struktur, Spinelle, Silikate, Kieselsäure sowie temperaturbeständige andere Oxide z. B. des Titans, Zirkons, Magnesiums und z. B. Carbonate von Erdalkalimetallen empfohlen, ferner Carbide, Kohlenstoff und natürlich vorkommende Trägermaterialien wie Bimsstein oder Kieselgur.

Nach der Erfahrung erfordert der Träger keine besondere Auswahl, jedoch haben sich Aluminiumoxide und künstliche Spinelle gut bewährt.

Die angewandten Mengen an Wirkmetallen sind wie üblich bei Edelmetallen gering, z. B. im Bereich von weniger als einigen Prozenten.

Die für eine Gasphasenreaktion übliche Technologie kann angewandt werden, z. B. eignen sich Rohrreaktoren, Wirbelbetten oder Schachtreaktoren, die auch die äußere Form des Katalysators bestimmen.

Die Reaktionstemperatur soll so niedrig wie möglich liegen, jedoch im allgemeinen mindestens 150° C sein. Man muß dabei beachten, daß die Manteltemperatur des Reaktionsraumes oftmals nicht der tatsächlich auf der Katalysatoroberfläche herrschenden Temperatur entspricht und diese daher möglichst innerhalb des Gasraumes gemessen werden sollte.

Gewöhnlich wird man bei atmosphärischem Druck oder in dessen Nähe arbeiten; wenn die jeweilige Siedetemperatur des Ausgangsmaterials noch erreicht wird, kann man mit etwas erhöhtem Druck eine bessere Ausnutzung des Reaktionsraumes erzielen.

Wasserstoff wird im Überschuß angewendet, der z. B. das 2- bis 100fache des rechnerisch erforderlichen betragen kann. Im allgemeinen ist ein 10facher Überschuß ausreichend.

Als Dinitrile sind zur Herstellung von Pyridinen Glutarsäurenitril und seine Substitutionsprodukte erforderlich. Sie können in jeder $CH_2$-Gruppe einfach substituiert sein; wirtschaftlich bedeutsam ist die Substitution durch eine kurze Alkylgruppe in 2- oder 3-Stellung, die zu $\beta$- oder $\gamma$-Picolin und seinen 3- oder 4-Alkylhomologen führt und damit den Weg zu Nicotin- bzw. Isonicotinsäure und deren Abkömmlingen vereinfacht.

Aus Bernsteinsäurenitrilen erhält man entsprechend Pyrrol und dessen Substitutionsprodukte in 3- und/oder 4-Stellung, besonders die Alkylderivate.

Die nach dem Durchlaufen der Reaktion durch Kondensation gewinnbaren Umsetzungsprodukte können außer dem jeweiligen Zielprodukt auch teilweise Umsetzungsprodukte, nämlich das entsprechende Diamin und das gesättigte cyclische Amin enthalten. Es versteht sich, daß diese gewöhnlich leicht abtrennbaren Verbindungen zurückgeführt werden können und ihre Entstehung somit keine Minderung der Ausbeute bzw. Selektivität der Umsetzung bedeutet.

Die Durchführbarkeit der Erfindung wurde u. a. mit den nachfolgenden Experimenten untersucht:

### Beispiel 1

Ein Rohr aus V2A-Stahl mit einem Durchmesser von 10 mm und einer Länge von 250 mm, das mit 200 ml eines Katalysators gefüllt ist, der 0,7% Pd auf $Al_2O_3$ enthält, wird pro Stunde mit 23 l Wasserstoff beschickt, in dem 23 g 2-Methylglutarsäuredinitril verdampft worden waren. Die Außentemperatur des Rohres beträgt 280° C.

Die Reaktionsprodukte werden kondensiert und gesammelt. Nach 10 Stunden haben sich 189 g Kondensat abgeschieden, die destilliert und gaschromatografisch untersucht werden. Man findet 184 g $\beta$-Picolin und 13 g 3-Methylpiperidin entsprechend einer Gesamtausbeute von 83%.

### Beispiel 2

Ein auf 250°C geheiztes Rohr aus V2A-Stahl mit einem Durchmesser von 10 mm und einer Länge von 50 mm, das mit 26 g eines Katalysators (0,35% Pt/Al$_2$O$_3$) gefüllt ist, wird innerhalb von 10 Stunden mit 85 g 2-Methylglutarsäuredinitril, verdampft in 180 l Wasserstoff beschickt.

Man erhält 76 g eines Gemisches, das aus 13% 3-Methylpiperidin, 50% $\beta$-Picolin, 0,2% 2-Methylglutardinitril und im übrigen nicht identifizierten Produkten besteht.

Bezogen auf das eingesetzte Dinitril und unter Berücksichtigung des Piperidins bedeutet dies eine Ausbeute von 70%.

### Beispiel 3

Einem Quarzrohr mit einem Durchmesser von 30 mm und einer Länge von 500 mm, das mit 220 ml eines Katalysators gefüllt ist, der 1% Palladium auf einem Lithium-Aluminium-Spinell enthält, werden pro Stunde 20 ml eines Gemisches aus 89% 2-Methylglutarsäuredinitril und 10% 2-Ethylbernsteinsäuredinitril, verdampft in 200 l Wasserstoff zugeführt. Die Außentemperatur des Quarzrohrs beträgt 250°C. Man kondensiert die Reaktionsprodukte und sammelt sie, bis 2023 g Dinitrilgemisch verbraucht sind. Es werden 1687 g Kondensat mit 40 Gew.-% $\beta$-Picolin und 37 Gew.-% Methylpiperidin erhalten. Dies entspricht einer Ausbeute von 81%, bezogen auf das eingesetzte 2-Methylglutarsäuredinitril.

### Beispiel 4

Wie in Beispiel 2 beschrieben, werden an 20 g eines Katalysators (0,5% Pd auf Al$_2$O$_3$) pro Stunde 2,5 g Glutarsäuredinitril und 10 l Wasserstoff bei einer Manteltemperatur von 250°C umgesetzt.

Nach 20 Stunden wird das erhaltene Kondensat untersucht. Man findet 20 g Pyridin und 12 g Piperidin. Ausbeute: 75%.

### Beispiel 5

Einem Wirbelbett von 1 l Katalysator (5% Palladium, 0,25% Selen auf Aktivkohle), das bei einer Temperatur von 310°C gehalten wird, werden 100 ml/h 2-Methylglutarsäuredinitril (90%ig) und 350 l/h Wasserstoff zugeführt.

Man findet 3-Methylpiperidin: 3,7, $\beta$-Picolin: 66%, 2-Methylglutardinitril: 11% sowie nicht identifizierte Verbindungen.

Die Selektivität, bezogen auf umgesetztes 2-Methylglutardinitril, beträgt 75%.

### Patentansprüche

1. Verfahren zur Herstellung von Pyridinen oder Pyrrolen aus $\alpha$, $\omega$-Dinitrilen gleicher Kohlenstoffzahl mit der Struktur eines ggf. substituierten Glutarsäure- bzw. Bernsteinsäuredinitrils, dadurch gekennzeichnet, daß man das Dinitril in der Gasphase an einem hydrierend wirkenden Edelmetallkatalysator mit überschüssigem Wasserstoff umsetzt.

2. Verfahren nach Anspruch 1, dadurch gekennzeichnet, daß man einen Platin oder Palladium enthaltenden Trägerkatalysator verwendet.

### Claims

1. A process for the preparation of a pyridine or a pyrrole from an $\alpha$, $\omega$-dinitrile which has the same number of carbon atoms and the structure of an unsubstituted or substituted glutarodinitrile or succinodinitrile, wherein the dinitrile is reacted in the gas phase with excess hydrogen, over a noble metal hydrogenation catalyst.

2. A process as claimed in claim 1, wherein a platinum-containing or palladium-containing supported catalyst is used.

### Revendications

1. Procédé de préparation de pyridines ou de pyrroles à partir de $\alpha$, $\omega$-dinitriles d'un même nombre d'atomes de carbone avec la structure d'un dinitrile succinique ou glutarique éventuellement substitué, caractérisé en ce que l'on fait réagir le dinitrile, en phase gazeuse, sur un catalyseur d'hydrogénation à métal noble avec de l'hydrogène en excès.

2. Procédé suivant la revendication 1, caractérisé en ce que l'on emploie un catalyseur au platine ou au palladium sur support.